(19) Europäisches Patentamt
European Patent Office
Office européen des brevets

(11) Publication number: **0 067 025**
**B1**

(12) **EUROPEAN PATENT SPECIFICATION**

(45) Date of publication of patent specification: **09.09.87**

(51) Int. Cl.⁴: **C 11 D 3/22,** C 11 D 3/37, A 61 K 7/50

(21) Application number: **82302803.0**

(22) Date of filing: **01.06.82**

(54) **Liquid cleansing product.**

(30) Priority: **04.06.81 US 270436**

(43) Date of publication of application:
**15.12.82 Bulletin 82/50**

(45) Publication of the grant of the patent:
**09.09.87 Bulletin 87/37**

(84) Designated Contracting States:
**BE DE FR GB IT NL**

(56) References cited:
**EP-A-0 034 190**
**EP-A-0 048 612**
**US-A-4 143 007**

**CHEMICAL ABSTRACTS, vol. 93, 1980, pages 90,91, *no.* 48451v, *Columbus, Ohio*, USA; & JP - A - 80 36371 (TOYOBO CO., LTD.) 13-03-1980**

(73) Proprietor: **THE PROCTER & GAMBLE COMPANY**
**301 East Sixth Street**
**Cincinnati Ohio 45202 (US)**

(72) Inventor: **Hoskins, James Jay**
**690 Yorkhaven Road**
**Cincinnati Ohio 45240 (US)**
Inventor: **Kessler, Adriaan**
**3674 Clifton Avenue**
**Cincinnati Ohio 45220 (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

**EP 0 067 025 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to liquid cleansing products and, more specifically, to liquid cleansing products which provide beneficial skin feel properties to the product.

Liquid cleansing products are extensively used as hand cleaners, shampoos, and for many other purposes. The current invention is concerned primarily with cleansers used for cleaning skin but is not limited to that application. The cleansers of interest are aqueous based and contain surfactant.

In order to achieve controlled use of a liquid cleansing product, it is desirable to have a viscous but pourable or pumpable product. A thin, watery product is too easily spilled and wasted when used and does not have good consumer acceptance. Guar gum and its chemically modified derivatives are well known product ingredients used in a wide variety of aqueous based products and are described variously as thickeners, gelling agents, suspending agents, protective colloids, binder materials, agglomerating agents, etc. As used herein, the term "guar material" is used to denote guar gum or hydroxypropyl guar gum.

In the present invention, guar materials have been found to be effective as thickening agents in liquid cleanser formulations. In addition, it has been found that guar material, and particularly certain derivatives of guar gum, imparts a desirable smooth, slippery skin feel to the liquid cleansing products. However, cleansing products formulated with the levels of guar material needed to provide the desired thickening and skin feel properties are not stable, but tend to separate. A thick phase, presumably high in guar material content, settles to the bottom of the product container. It was found that this separation can be eliminated by incorporating a substantial level of alcohol (greater than 4%) in the formulation. This level of alcohol in the liquid cleansing product is undesirable because it is believed to have a tendency to dry the skin when used. Another solution to the settling problem is therefore necessary. The use of carboxyvinyl polymer represents such a solution.

Carboxyvinyl polymers are a family of acrylic acid copolymers marketed by the B. F. Goodrich Company, New York, N.Y. under the tradename of Carbopol (RTM). Carboxyvinyl polymers are well known ingredients that impart many of the same functional benefits as guar materials to aqueous based compositions. In fact, carboxyvinyl polymers and guar materials are often indicated as being interchangeable ingredients in particular formulations. For example, in US—A—3,697,644, carboxypolymethylene (a carboxyvinyl polymer, Carbopol (RTM) 934) and guar gum (a guar material, Jaguar (RTM) A-20-D from Stein, Hall and Company) are specified as alternative thickening agents which act as a protective colloid to hold a dispersed phase in suspension in cosmetic compositions. In US—A—3,939,260 Carbopol (RTM) and guar gum are used alternatively as a thickener or binder in therapeutic and cosmetic compositions.

In US—A—3,591,680, the use of Carbopol (RTM) or guar gum as a suspending agent in antacid compositions is disclosed. Guar gum or Carbopol (RTM) can be used as a binder material in producing a battery gel substance in US—A—3,880,672. The document considers guar gum and carboxyvinyl polymer to be different materials that can be used interchangeably for the same purpose along with a number of other materials. These references regard the individual materials or any combination of them to be the same. Neither reference specifically discloses a combination of carboxyvinyl polymer and guar gum, and neither suggests that such a combination might have more desirable properties than either used separately in a liquid skin cleanser.

It is therefore an object of the present invention to provide liquid cleansing compositions which deliver good skin feel and are stable.

It is a further object of the present invention to provide liquid cleansing compositions which utilize particular levels of a guar material and a carboxyvinyl polymer.

These and other objects will become apparent from the following detailed description. All percentages herein are by weight unless otherwise specified.

## Summary of the invention

The invention described herein is a liquid cleansing product comprising from 5 to 30% of surfactant, from 0.1 to 1.0% of guar gum material selected from guar gum and hydroxypropyl guar gum, from 0.15 to 1.0% of carboxyvinyl polymer as hereinbelow defined, and water.

## Detailed description of the invention

This invention relates to liquid cleansing products that utilize a combination of guar material and carboxyvinyl polymer to achieve a shelf stable, thickened product with superior skin feel characteristics. The liquid cleansing products of this invention are aqueous based formulations containing surfactant, guar material, and carboxyvinyl polymer. Preferred liquid cleansing products also contain, as minor ingredients, compounds which function as opacifiers, perfumes, colorants, preservatives, and pH adjusters. The necessary and optional ingredients are described in detail below.

### Guar material

In the present liquid cleansing products it has been found that guar material can provide a highly desirable smooth, slippery skin feel to the products. Guar gum is a naturally occurring material which is the principal component of the seed of the guar plant. Guar gum is extracted from the guar seed and purified.

Guar gum is a high molecular weight carbohydrate polymer or polysaccharide made up of mannose and galactose units linked together. The guar molecule is essentially a straight chain of mannose units linked to each other by means of beta (1—4) glycosidic linkages. Galactose units branch from alternate mannose units through alpha (1—6) linkages with the mannose units.

The desired skin feel of the liquid cleansing products is preferably obtained by using hydroxypropyl guar gum. In the guar gum molecule, each mannose and galactose unit has from 2—4 hydroxyl groups depending on where it is located in the polymer chain. Guar gum derivatives are produced by reacting guar gum such that substitution of chemical moieties occurs on some of these hydroxyl units. Hydroxypropyl guar gums are a family of materials with hydroxypropyl groups substituted for some of the hydroxyl units. The term "degree of substitution" is used to indicate the average number of hydroxypropyl units which occur on each of the sugar units in the polymer molecule. It is preferred that the hydroxypropyl guar gum used in the present invention have a degree of substitution of 0.3 to 1.2; especially preferred is hydroxypropyl guar gum with a degree of substitution of about 0.6. Such a material is available commercially as Jaguar (RTM) HP-60 from Celanese Plastics & Specialties Company.

The quantity of guar material used in the liquid cleansing products of this invention is from 0.1% to 1.0%. A preferred range of usage for hydroxypropyl guar gum is from 0.2% to 0.5%; especially preferred is about 0.3%. A preferred range of usage for guar gum is from 0.4% to 0.7%.

Carboxyvinyl polymer

Although guar material alone will provide the desired skin feel in liquid cleansing products, it was found that such products are not shelf stable. In time, a viscous layer, presumably high in guar material content, settles to the bottom of the product container. It was found that the addition of carboxyvinyl polymer as herein defined to the liquid cleansing products provided shelf stable products while retaining the desired superior skin feel properties. The carboxyvinyl polymer is defined herein as a water-soluble polymer of acrylic acid cross-linked with about 1% of a polyallyl ether of sucrose having an average of about 5.8 allyl groups for each sucrose molecule.

A preferred carboxyvinyl polymer for the liquid cleansing products is Carbopol (RTM) 940; especially preferred is Carbopol (RTM) 934. Both are commercially available from B. F. Goodrich Company, New York, N.Y. Carbopol (RTM) 934 is a very slightly cross-linked carboxyvinyl polymer of extremely high molecular weight having an acid equivalent weight of about 73 to 76 and is capable, when at least partially neutralized, of greatly increasing the viscosity of aqueous systems. Carbopol (RTM) 940 is a similarly constituted product. Carboxyvinyl polymers and methods for making them are described in US—A—2,798,053.

The quantity of carboxyvinyl polymer used in the liquid cleansing products of this invention is from 0.15% to 1.0%. A preferred range of usage is from 0.2 to 0.5%; especially preferred is about 0.35%.

Surfactant

The liquid cleansing products of this invention contain a surfactant system comprising one or more surfactants.

The surfactants suitable herein are water soluble foaming organic detergents selected from anionic, nonionic, cationic, zwitterionic and amphoteric classes. Suitable detergent materials are those which provide copious suds formation and cleansing properties. Examples of surfactant materials from which the liquid cleansing products of the invention can be selected include the water soluble anionic, nonionic, cationic, zwitterionic and amphoteric detergents described as follows:

a. Anionic detergents include the synthetic non-soap detergents which can be broadly described as the water-soluble salts, particularly the alkali metal and ammonium salts, of organic sulfuric reaction products having in their molecular structure an alkyl radical containing from 8 to 22 carbon atoms and a radical selected from the group consisting of sulfonic acid and sulfuric acid ester radicals. (Included in the term alkyl is the alkyl portion of higher acyl radicals.) Important examples of the synthetic detergents which form a part of the compositions of the present invention are the alkali metal, e.g. sodium or potassium, and ammonium alkyl sulfates, especially those obtained by sulfating the higher alcohols ($C_8$—$C_{18}$ carbon atoms) produced by reducing the glycerides of tallow or coconut oil; the alkali metal olefin sulfonates of from 8 to 24 carbon atoms described, for example, in US—A—3,332,880, and the alkali metal alkyl glyceryl ether sulfonates, especially those ethers of the higher alcohols derived from tallow and coconut oil; other anionic detergents include the alkali metal alkylbenzene sulfonates, in which the alkyl group contains from 9 to 15 carbon atoms, including those of the types described in US—A—2,220,099 and US—A—2,477,383 (the alkyl radical can be a straight or branched aliphatic chain); sodium coconut oil fatty acid monoglyceride sulfates and sulfonates; sodium, potassium, or ammonium salts of sulfuric acid esters of the reaction product of one mole of a higher fatty alcohol (e.g., tallow or coconut oil alcohols) and 1 to 12 moles of ethylene oxide (hereinafter referred to as "alkali metal or ammonium alkyl ethoxy sulfates"); sodium or potassium salts of alkyl phenol ethylene oxide ether sulfate with 1 to 10 units of ethylene oxide per molecule and in which the alkyl radicals contain from 8 to 12 carbon atoms; the reaction product of fatty acids esterified with isethionic acid and neutralized with sodium hydroxide where, for example, the fatty acid is oleic or derived from coconut oil; sodium or potassium salts of fatty acid amide of a methyl tauride in which the fatty acids, for example, are derived from coconut oil, sodium or potassium β-acetoxy- or

β-acetamidoalkane-sulfonates where the alkane has from 8 to 22 carbon atoms; and others known in the art, a number specifically set forth in US—A—2,486,921, US—A—2,486,922 and US—A—2,396,278.

b. Nonionic synthetic detergents: One class can be broadly defined as compounds produced by the condensation of alkylene oxide groups (hydrophilic in nature) with an organic hydrophobic compound, which can be aliphatic or alkyl aromatic in nature. The length of the hydrophilic or polyoxyalkylene radical which is condensed with any particular hydrophobic group can be readily adjusted to yield a water-soluble compound having the desired degree of balance between hydrophilic and hydrophobic elements. Another class has semi-polar characteristics. Classes of nonionic synthetic detergents are as follows:

1. The monoethanol, diethanol, and ammonia amides of fatty acids having an acyl moiety of from 8 to 18 carbon atoms. These acyl moieties are normally derived from naturally occurring glycerides, e.g., coconut oil, palm oil, soybean oil and tallow, but can be derived synthetically, e.g., by the oxidation of petroleum, or by hydrogenation of carbon monoxide by the Fischer-Tropsch process.

2. A class of nonionic synthetic detergents under the trade name of "pluronic (RTM)". These compounds are formed by condensing ethylene oxide with a hydrophobic base formed by the condensation of propylene oxide with propylene glycol. The hydrophobic portion of the molecule which, of course, exhibits water insolubility, has a molecular weight of from 1500 to 1800. The addition of polyoxyethylene radicals to this hydrophobic portion tends to increase the water solubility of the molecule as a whole and the liquid character of the product is retained up to the point where the polyethylene content is about 50% of the total weight of the condensation product.

3. The polyethylene oxide condensates of alkyl phenols, e.g., the condensation products of alkyl phenols having an alkyl group containing from 6 to 12 carbon atoms in either a straight chain or branched chain configuration with ethylene oxide, the said ethylene oxide being present in amounts equal to 5 to 25 moles of ethylene oxide per mole of alkyl phenol. The alkyl substituent in such compounds may be derived from polymerized propylene, diisobutylene, octene, or nonene, for example.

4. Those nonionic synthetic detergents derived from the condensation of ethylene oxide with the product resulting from the reaction of propylene oxide and ethylene diamine. For example, compounds containing from 40 to 80% polyoxyethylene by weight and having a molecular weight of from 5,000 to 11,000 resulting from the reaction of ethylene oxide groups with a hydrophobic base constituted of the reaction product of ethylene diamine and excess propylene oxide; said base having a molecular weight of the order of 2,500 to 3,000 are satisfactory.

5. The condensation product of aliphatic alcohols having from 8 to 22 carbon atoms, in either straight chain or branched chain configuration, with ethylene oxide, e.g., a coconut alcohol ethylene oxide condensate having from 5 to 30 moles of ethylene oxide per mole of coconut alcohol, the coconut alcohol fraction having from 10 to 14 carbon atoms.

6. Long chain tertiary amine oxides corresponding to the following general formula

$$R^1—(OR^4)_n—\overset{\displaystyle R^2}{\overset{|}{\underset{\underset{\displaystyle R^3}{|}}{N}}}\rightarrow O$$

wherein $R^1$ is an alkyl radical of from 8 to 24 carbon atoms, $R^2$ and $R^3$ are each methyl, ethyl or hydroxyethyl radicals, $R^4$ is ethylene, and n equals from 0 to 10. The arrow in the formula is a conventional representation of a semi-polar bond. Specific examples of amine oxide detergents include: dimethyldodecylamine oxide; cetyldimethylamine oxide; bis-(2-hydroxyethyl) dodecylamine oxide; and bis-(2-hydroxyethyl)-3-dodecoxy-1-hydroxypropyl amine oxide.

7. Long chain tertiary phosphine oxides, corresponding to the following general formula $RR'R''P\rightarrow O$ wherein R is an alkyl, alkenyl or monohydroxyalkyl radical ranging from 10 to 24 carbon atoms in chain length an R' and R'' are each alkyl or monohydroxyalkyl groups containing from 1 to 3 carbon atoms. The arrow in the formula is a conventional representation of a semi-polar bond. Examples of suitable phosphine oxides are found in US—A—3,304,262 and include: dimethyldodecylphosphine oxide; diethyldodecylphosphine oxide; dimethyl-(2-hydroxydodecyl) phosphine oxide.

8. Long chain sulfoxides having the formula

$$R^5—\overset{\displaystyle O}{\overset{\uparrow}{S}}—R^6$$

wherein $R^5$ is an alkyl radical containing from 10 to 28 carbon atoms, from 0 to 5 ether linkages and from 0 to 2 hydroxyl substituents, at least one moiety of $R^5$ being an alkyl radical containing 0 ether linkages and containing from 10 to 18 carbon atoms, and wherein $R^6$ is an alkyl radical containing from 1 to 3 carbon atoms and from one to two hydroxyl groups. Specific examples of these sulfoxides are: dodecyl methyl sulfoxide; 3-hydroxy tridecyl methyl sulfoxide; 3-methoxy tridecyl methyl sulfoxide; and 3-hydroxy-4-dodecoxybutyl methyl sulfoxide.

c. Amphoteric synthetic detergents can be broadly described as derivatives of aliphatic secondary and tertiary amines, in which the aliphatic radical may be straight chain or branched and wherein one of the aliphatic substituents contains from 8 to 18 carbon atoms and one contains an anionic water solubilizing group, e.g., carboxy, sulfo, sulfato, phosphato, or phosphono. Examples of compounds falling within this definition are sodium-3-dodecylaminopropionate and sodium-3-dodecylaminopropane sulfonate.

d. Zwitterionic synthetic detergents can be broadly described as derivatives of aliphatic quaternary ammonium, phosphonium, and sulfonium compounds, in which the aliphatic radical may be straight chain or branched, and wherein one of the aliphatic substituents contains from 8 to 18 carbon atoms and one contains an anionic water solubilizing group, e.g., carboxy, sulfo, sulfato, phosphato, or phosphono. Examples of compounds falling within this definition are 3-(N,N-dimethyl-N-hexadecylammonio) propane-1-sulfonate and 3-(N,N-dimethyl-N-hexadecylammonio)-2-hydroxy propane-1-sulfonate.

e. Cationic detergents include those having the formula $R—N(R^2)_3^{(+)}X^{(-)}$ wherein R is an alkyl chain containing from 8 to 20 carbon atoms, each $R^2$ is selected from the group consisting of alkyl and alkanol groups containing from 1 to 4 carbon atoms and benzyl groups there being normally no more than one benzyl group and two $R^2$ groups can be joined by either a carbon-carbon ether, or imino linkage to form a ring structure, and X represents a halogen atom, sulfate group, nitrate group or other pseudohalogen group. Specific examples are coconut alkyl trimethyl amine chloride, dodecyl dimethyl benzyl bromide, and dodecyl methyl morpholino chloride.

A water-soluble surfactant is employed in the invention in an amount sufficient to provide desired suds formation and cleaning under normal usage conditions. In some instances copious suds formation will be desired while in others a composition providing less sudsing may be preferred by the user. Depending upon the foaming capacity and amounts of the particular detergents employed, desirable variations can be obtained. Normally the surfactant will comprise from 5% to 30% by weight of the composition. Preferably, the surfactant comprises from 10% to 25% by weight of the composition; especially preferred is about 15—20% by weight surfactant in the liquid cleansing products.

Preferred surfactants for use in the liquid cleansing products include the alkali metal or ammonium alkyl sulfates, alkali metal or ammonium alkyl ethoxy sulfates, and monoethanol and diethanol amides of fatty acids described hereinabove, and mixtures thereof. Especially preferred is a mixture of sodium alkyl sulfate, sodium alkyl ethoxy (3) sulfate, and coconut monoethanol amide.

Optional ingredients

Where the composition of a liquid cleansing product will be in contact with the skin of the users, it is preferred that the cleansing product be formulated to provide a pH in use within the range of from 4 to 10, depending upon the particular surfactant or materials employed. Especially preferred is a pH in the range of 6 to 7. Any of a large number of known substances can be used to adjust the pH of the liquid cleansing product, e.g., sodium hydroxide, citric acid, generally at a level of up to 0.5% of the product composition.

Materials that provide skin conditioning benefits such as humectants, e.g., glycerin, may be added to the liquid cleansing products of the present invention, generally at a level of 1% to 8% of the product composition. Perfumes may be used in formulating the liquid cleansing products, generally at a level of 0.1% to 1.0% of the product composition. Colorants may also be used in the liquid cleansing products. Opacifiers, e.g., ethylene glycol distearate, polystyrene latex, generally at a level of 0.2% to 2.0% of the product composition, may be used in the liquid cleansing products to provide them with an opaque or pearlescent appearance. Preservatives, e.g., EDTA, methyl paraben, propyl paraben, Germall (RTM) 115, Kathon (RTM), generally at a level of less than 1%, may be incorporated in the liquid cleansing products to prevent microbiological growth in the products.

The liquid cleansing products of the present invention contain from 60% to 95% water, preferably from 70% to 90% water.

Method of manufacture

A method of making liquid cleansing products of the present invention is described in Example 1 which follows.

Industrial applicability

The liquid cleansing products of the present invention are designed primarily for the cleaning of human skin; they are expected to be used especially for the washing of hands.

The following examples will illustrate the invention, but are not intended to be any way limiting thereof.

Example 1

| Component | Amount |
|---|---|
| sodium $C_8$ to $C_{18}$ alkyl sulfate | 7.2% |
| sodium $C_8$ to $C_{18}$ alkyl ethoxy (3) sulfate | 8.8% |
| coconut monoethanol amide | 4.0% |
| Jaguar (RTM) HP-60 | 0.30% |
| Carbopol (RTM) 934 | 0.35% |
| perfumes, colorants, preservatives, opacifiers and pH adjusters | less than 3% |
| water | balance |

A liquid cleansing product of the above composition was produced by a batch process comprising the following steps:

1. A Carbopol (RTM) premix solution was prepared by adding the Carbopol (RTM) slowly to water being agitated. After the Carbopol (RTM) was dissolved, the solution was heated to about (57°C) (135°F) to (65°C) (150°F).

2. Water was added to the batch mix tank and the anionic surfactants were added to it. The solution was heated to about (65°C) (150°F).

3. Preservatives and nonionic surfactant were added to the batch mix tank with agitation.

4. A guar material premix solution was prepared by adding the guar material to water that had been made alkaline by the addition of a small quantity of sodium hydroxide.

5. The guar material solution was added to the batch mix tank with agitation such that good dispersion of the guar material solution was assured.

6. The colorant, the Carbopol (RTM) solution, and then the opacifier were added to the batch mix tank with agitation.

7. The contents of the batch mix tank were cooled to below (57°C) (135°F), the perfume was added, and the pH was adjusted to between 6 and 7 by the addition of a small quantity of citric acid or sodium hydroxide. Cooling was continued until the product temperature was below (32°C). (90°F)

Example 2

| Component | Amount |
|---|---|
| sodium $C_8$ to $C_{18}$ alkyl sulfate | 8.0% |
| sodium $C_8$ to $C_{18}$ alkyl ethoxy (12) sulfate | 8.0% |
| coconut monoethanol amide | 2.0% |
| glycerine | 2.0% |
| Jaguar (RTM) HP-11* | 0.30% |
| Carbopol (RTM) 934 | 0.30% |
| preservatives, opacifiers, colorants, perfumes, and pH adjusters | less than 3% |
| water | balance |

* Hydroxypropyl guar gum with a degree of substitution of about 0.3 from Celanese Plastics & Specialties Company.

A liquid cleansing product is made with the composition above using the process described in Example 1. Glycerine is added to the batch mix tank in step 3.

Example 3

| Component | Amount |
|---|---|
| ammonium $C_8$ to $C_{18}$ alkyl sulfate | 5.0% |
| ammonium $C_8$ to $C_{18}$ alkyl ethoxy (3) sulfate | 7.8% |
| coconut monoethanol amide | 4.0% |
| Jaguar (RTM) HP-60 | 0.35% |
| Carbopol (RTM) 934 | 0.35% |
| perfumes, colorants, preservatives, opacifiers and pH adjusters | less than 3% |
| water | balance |

A liquid cleansing product of the above composition is made by the process described in Example 1.

Example 4

| Component | Amount |
|---|---|
| ammonium $C_8$ to $C_{18}$ alkyl sulfate | 5.5% |
| ammonium $C_8$ to $C_{18}$ alkyl ethoxy (3) sulfate | 5.6% |
| coconut monoethanol amide | 1.0% |
| coconut fatty acid | 1.0% |
| Jaguar (RTM) HP-60 | 0.30% |
| Carbopol (RTM) 934 | 0.25% |
| perfumes, colorants, preservatives, opacifiers and pH adjusters | less than 3% |
| water | balance |

A liquid cleansing product of the above composition is made by the process described in Example 1.

Example 5

| Component | Amount |
|---|---|
| sodium $C_8$ to $C_{18}$ alkyl sulfate | 7.2% |
| sodium $C_8$ to $C_{18}$ alkyl ethoxy (3) sulfate | 8.8% |
| coconut monoethanol amide | 4.0% |
| Jaguar (RTM) A-40-F* | 0.60% |
| Carbopol (RTM) 934 | 0.35% |
| perfumes, colorants, preservatives, opacifiers and pH adjusters | less than 3% |
| water | balance |

* Purified guar gum from Celanese Plastics & Specialties Company.

A liquid cleansing product of the above composition is made by the process described in Example 1.

**Claims**

1. A liquid cleansing product comprising:
(a) from 5% to 30% of surfactant;
(b) from 0.1% to 1.0% of guar gum material selected from guar gum and hydroxypropyl guar gum;
(c) from 0.15% to 1.0% of carboxyvinyl polymer, said carboxyvinyl polymer being a water-soluble polymer of acrylic acid cross-linked with about 1% of a polyallyl ether of sucrose having an average of about 5.8 allyl groups for each sucrose molecule; and
(d) water.

2. A liquid cleansing product according to Claim 1 wherein the guar gum material is hydroxypropyl guar gum.

3. A liquid cleansing product according to Claim 2 wherein the hydroxypropyl guar gum has a degree of substitution of from 0.3 to 1.2, preferably about 0.6.

4. A liquid cleansing product according to any of Claims 1 to 3 wherein said surfactant is selected from anionic and nonionic detergents, and mixtures thereof.

5. A liquid cleansing product according to any of Claims 1 to 4 wherein said surfactant is selected from alkali metal and ammonium alkyl sulfates, alkali metal and ammonium alkyl ethoxy sulfates, and the monoethanol and diethanol amides of fatty acids, and mixtures thereof.

6. A liquid cleansing product according to any of Claims 1 to 5 wherein said surfactant is selected from sodium alkyl sulfate, sodium alkyl ethoxy (3) sulfate, and coconut monoethanol amide, and mixtures thereof.

7. A liquid cleansing product according to any of Claims 1 to 6 wherein said cleansing product comprises from 10% to 25% of surfactant, from 0.2% to 0.5% of hydroxypropyl guar gum, and from 0.2% to 0.5% of carboxyvinyl polymer.

8. A liquid cleansing product according to Claim 7 wherein said cleansing product comprises from 15% to 20% of surfactant, about 0.3% of hydroxypropyl guar gum, and about 0.35% of carboxyvinyl polymer.

**Patentansprüche**

1. Flüssiges Reinigungsmittel, enthaltend:
(a) 5 bis 30% eines oberflächenaktiven Mittels;
(b) 0,1 bis 1,0% eines Guargummimaterials, ausgewählt aus Guargummi und Hydroxypropylguargummi;
(c) 0,15 bis 1,0% eines Carboxyvinylpolymeren, wobei das Carboxyvinylpolymer ein wasserlösliches Polymer von Acrylsäure, vernetzt mit etwa 1% eines Polyallylethers von Saccharose mit durchschnittlich etwa 5,8 Allylgruppen für jedes Saccharosemolekül ist; und
(d) Wasser.

2. Flüssiges Reinigungsmittel gemäss Anspruch 1, worin das Guargummimaterial Hydroxypropylguargummi ist.

3. Flüssiges Reinigungsmittel gemäss Anspruch 2, worin das Hydroxypropylguargummi einen Substitutionsgrad von 0,3 bis 1,2, vorzugsweise von etwa 0,6-aufweist.

4. Flüssiges Reinigungsmittel gemäss einem der Ansprüche 1—3, worin das oberflächenaktive Mittel ausgewählt ist aus anionischen und nichtionischen Detergentien und Gemischen daraus.

5. Flüssiges Reinigungsmittel gemäss einem der Ansprüche 1—4, worin das oberflächenaktive Mittel ausgewählt ist aus Alkalimetall- und Ammoniumalkylsulfaten, Alkalimetall- und Ammoniumalkyl-ethoxysulfaten und den Monoethanol- und Diethanolamiden von Fettsäuren und Gemischen daraus.

6. Flüssiges Reinigungsmittel gemäss einem der Ansprüche 1—5, worin das oberflächenaktive Mittel ausgewählt ist aus Natriumalkylsulfat, Natriumalkyl-ethoxy(3)-sulfat und Kokosnussmonoethanolamid und Gemischen daraus.

7. Flüssiges Reinigungsmittel gemäss einem der Ansprüche 1—6, worin das Reinigungsmittel 10 bis 25% des oberflächenaktiven Mittels, 0,2 bis 0,5% Hydroxypropylguargummi und 0,2 bis 0,5% Carboxyvinylpolymer enthält.

8. Flüssiges Reinigungsmittel gemäss Anspruch 7, worin das Reinigungsmittel 15 bis 20% des oberflächenaktiven Mittels, etwa 0,3% Hydroxypropylguargummi und etwa 0,35% Carboxyvinylpolymer enthält.

**Revendications**

1. Produit de nettoyage liquide, comprenant

(a) de 5 à 30% d'un tensioactif;

(b) de 0,1 à 1,0% d'une matière gomme de guar, choisie entre la gomme de guar et la gomme d'hydroxypropylguar;

(c) de 0,15 à 1,0% d'un polymère carboxyvinylique, ledit polymère carboxyvinylique étant un polymère d'acide acrylique soluble dans l'eau, réticulé avec environ 1,0% d'un éther polyallylique de saccharose, ayant en moyenne environ 5,8 groupes allyle par molécule de saccharose; et

(d) de l'eau.

2. Produit de nettoyage liquide selon la revendication 1, dans lequel la matière gomme de guar est la gomme d'hydroxypropylguar.

3. Produit de nettoyage liquide selon la revendication 2, dans lequel la gomme d'hydroxypropylguar présente un degré de substitution de 0,3 à 1,2, de préférence d'environ 0,6.

4. Produit de nettoyage liquide selon l'une quelconque des revendications 1 à 3, dans lequel ledit tensioactif est choisi parmi les détergents anioniques et non-ioniques, et leurs mélanges.

5. Produit de nettoyage liquide selon l'une quelconque des revendications 1 à 4, dans lequel ledit tensioactif est choisi parmi les alkylsulfates de métaux alcalins et d'ammonium, les alkyléthoxysulfates de métaux alcalins et d'ammonium, et le monoéthanolamide et le diéthanolamide d'acides gras, et leurs mélanges.

6. Produit de nettoyage liquide selon l'une quelconque des revendications 1 à 5, dans lequel ledit tensioactif est choisi entre les alkylsulfates de sodium, les alkyléthoxy(3)-sulfates de sodium et le (huile de coprah)-monoéthanolamide, et leurs mélanges.

7. Produit de nettoyage liquide selon l'une quelconque des revendications 1 à 6, dans lequel ledit produit de nettoyage comprend de 10 à 25% de tensioactif, de 0,2 à 0,5% de gomme d'hydroxypropylguar et de 0,2 à 0,5% de polymère carboxyvinylique.

8. Produit de nettoyage liquide selon la revendication 7, dans lequel ledit produit de nettoyage comprend de 15 à 20% de tensioactif, environ 0,3% de gomme d'hydroxypropylguar et environ 0,35% de polymère carboxyvinylique.